# EUROPEAN PATENT APPLICATION

(11) **EP 1 410 806 A1**
(43) Date of publication of application: **21.04.2004**
(21) Application number: 02751683.0
(22) Date of filing: 25.07.2002
(51) Int. Cl.: A61K 39/395, A61K 45/00, A61P 35/00

(54) **REMEDIES FOR MAMMARY CANCER**

(30) Priority: 25.07.2001 JP 2001224596
(71) Applicant: Mitsubishi Pharma Corporation, Osaka-shi, Osaka 541-0046 (JP)
(72) Inventor: HOSOKAWA, Saiko, c/o MITSUBISHI PHARMA CORP., Tokyo 103-8405 (JP); NIKI, Hisae, c/o MITSUBISHI PHARMA CORP., Tokyo 103-8405 (JP)
(74) Representative: TER MEER STEINMEISTER & PARTNER GbR
(86) International application number: PCT/JP2002/007548
(87) International publication number: WO 2003/009870

(57) **Abstract**

Provided is a remedy for cancer which remedy is specific to cancer tissue and has therapeutic effects on mammary cancer. The remedy is available by associating an antitumor substance with a human monoclonal antibody having amino acid sequences of SEQ. ID. NOS. 1, 2 and 3 of Sequence Listing in hypervariable regions of a heavy chain and amino acid sequences of SEQ. ID. NOS. 4, 5 and 6 of Sequence Listing in hypervariable regions of a light chain by attaching the antibody to a liposome having the antitumor substance encapsulated therein.

## Description

### TECHNICAL FIELD

The present invention relates to remedies for mammary cancer.

### BACKGROUND ART

A method of administering a medicament such as antitumor substance as a complex with an antibody by making use of the specific reactivity of the antibody and thereby accumulating the antitumor substance in cancer tissues has been developed. For example, an antibody-bound liposome, that is, a liposome having a medicament encapsulated therein and an antibody bound to the surface of the liposome is proposed as means for carrying a large amount of the medicament without modification has been proposed and excellent antitumor effects of it have been reported (Konno, et al., Cancer Research, 47, 4471 (1987), Hashimoto, et al, Japanese Patent Application Laid-Open No. Sho 58-13404).

As an antibody against cancer tissues, a GAH antibody which is a human monoclonal antibody screened for reactivity with gastric cancer and colorectal cancer is known (Japanese Patent Application Laid-Open No. Hei 4-346918 and Japanese Patent Application Laid-Open No. Hei 5-304987). Antibodies generally have markedly high specificity to antigens so that it is difficult even for those skilled in the art to forecast the reactivity of the GAH antibody, which has been screened for the reactivity with gastric cancer and lower bowel cancer, with another cancer.

As an antibody drug targeting to mammary cancer, an.antibody (refer to International Publication W089/6692) against HER2 (human epidermal growth factor receptor 2) is developed now, but this antibody is originally a mouse-derived monoclonal antibody and is humanized by genetic recombination so that its hypervariable region is derived from mouse. When an antibody is obtained by immunizing a known antigen to a mouse, it is easy to identify the cancer type by studying the distribution of the antigen itself by, for example, in situ hybridization, but in the case of a purely human-derived monoclonal antibody, it is difficult to know the distribution of the antigen itself or identify the cancer type, different from the mouse-derived antibody.

### DISCLOSURE OF THE INVENTION

An object of the present invention is to provide a cancer remedy specific to cancer tissues and effective for cancers including mammary cancer.

The present inventors have found that a GAH antibody has reactivity with mammary cancer as well as cancers of digestive tracts such as gastric cancer and colorectal cancer and thus has broad specificity; and this antibody associated with an antitumor substance effectively suppresses proliferation of mammary cancer cells and completed the present invention.

The present invention will next be summarized.
(1) A remedy for mammary cancer, which comprises a human monoclonal antibody having amino acid sequences of SEQ. ID. NOS. 1, 2 and 3 of Sequence Listing in hypervariable regions of a heavy chain and amino acid sequences of SEQ. ID. NOS. 4, 5 and 6 of Sequence Listing in hypervariable regions of a light chain; and an antitumor substance associated with the antibody.
(2) A remedy for mammary cancer as described above, wherein the monoclonal antibody has a heavy chain variable region containing an amino acid sequence of SEQ. ID No. 7 of Sequence Listing and a light chain variable region containing an amino acid sequence of SEQ. ID No. 8 of Sequence Listing.
(3) A remedy for mammary cancer as described above, wherein the antitumor substance has been associated with the antibody by binding the antibody to the surface of a liposome having the antitumor substance encapsulated therein.
(4) A remedy for mammary cancer as described above, wherein the antibody has been bound to the surface of the liposome by attaching via a thioether group the antibody to the liposome having a lipid end partially maleimidated.
(5) A remedy for mammary cancer as described above, wherein 0.1 to 2 mole% of the antibody has been bound to 1 mole of the maleimidated lipid.
(6) A remedy for mammary cancer as described above, wherein the antibody has been bound to the surface of the liposome by causing the maleimide-containing liposome to react with a sulfur-containing group derived from the antibody to form a thioether bond.
(7) A remedy for mammary cancer as described above, wherein to the surface of the liposome, a compound containing a polyalkyleneglycol portion has been bound further.
(8) A remedy for mammary cancer as described above, wherein 15 to 50 mole% of the compound containing a polyalkyleneglycol portion has been bound to 1 mole of the maleimidated lipid contained in the liposome.
(9) A remedy for mammary cancer as described above, wherein the compound containing a polyalkyleneglycol portion has been bound to the surface of the liposome by causing the maleimide group of the maleimidated lipid to react with the compound containing a polyalkyleneglycol portion added with a thiol group.
(10) A remedy for mammary cancer as described above, wherein the polyalkyleneglycol portion is a polyethyleneglycol portion.
(11) A remedy for mammary cancer as described above, wherein the compound containing a polyalkyleneglycol portion has two polyethyleneglycol portions.
(12) A remedy for mammary cancer as described above, wherein the polyethyleneglycol portion has a molecular weight of from 2,000 to 7,000 Daltons.
(13) A remedy for mammary cancer as described above, wherein the antibody is an F(ab')₂ fragment.

### BRIEF DESCRIPTION OF THE DRAWING

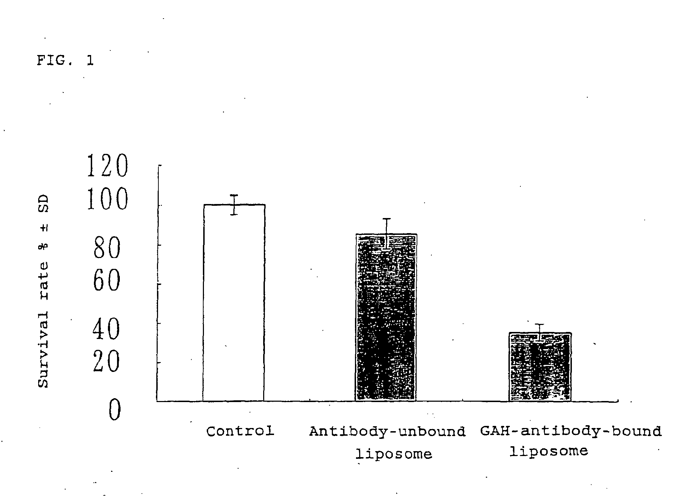

FIG. 1 illustrates the investigation results of the effects of a GAH antibody on the proliferation inhibition of cancer cell lines.

### BEST MODE FOR CARRYING OUT THE INVENTION

The remedy for mammary cancer according to the present invention comprises a human monoclonal antibody having amino acid sequences of SEQ. ID. NOS. 1, 2 and 3 of Sequence Listing in hypervariable regions of a heavy chain and having amino acid sequences of SEQ. ID. NOS. 4, 5 and 6 of Sequence Listing in hypervariable regions of a light chain; and an antitumor substance associated with the antibody.

The term "remedy for mammary cancer" as used herein means an antitumor agent for mammary cancer containing cells or tissues with which an antibody contained in the remedy shows reactivity.

The present invention has been completed based on the finding that a GAH antibody has reactivity with mammary cancer tissues and another finding that proliferation of mammary cancer can be suppressed effectively by this antibody associated with an antitumor substance. In the GAH antibody, regions of the amino acid sequences of SEQ. ID NOS. 1, 2 and 3 of Sequence Listing are called hypervariable regions, among heavy chain variable regions, and regions of the amino acid sequences of SEQ. ID NOS. 4, 5 and 6 are called hypervariable regions among light chain variable regions. Such regions determine the specificity of immunoglobulin as an antibody and binding affinity between an antigenic determinant and antibody and they are also called "complementarity determining regions". Regions other than such hypervariable regions therefore may be derived from another antibody. In other words, an antibody having hypervariable regions similar to those of a GAH antibody can also be used in the present invention.

Accordingly, the human monoclonal antibody to be used in the present invention has amino acid sequences of SEQ. ID NOS. 1, 2 and 3 of Sequence Listing in heavy chain hypervariable regions and amino acid sequences of SEQ. ID NOS. 4, 5 and 6 of Sequence Listing in light chain hypervariable regions. These amino acid sequences are usually contained in three hypervariable regions of the heavy chain and light chain in the order of SEQ. ID NOS. 1, 2 and 3 of Sequence Listing and in the order of SEQ. ID NOS. 4, 5 and 6 of Sequence Listing, respectively from the N-terminal side. In the monoclonal antibody usable in the present invention, those modified by, for example, substitution, insertion, deletion or addition of some amino acids within a range not impairing the reactivity with mammary cancer tissues are also embraced.

The human monoclonal antibody to be used in the present invention is available by forming a hybridoma between a lymphocyte derived from a cancer patient and a mouse myeloma cell and selecting the hybridoma having the above-described specific amino acid sequences.

The hybridoma is prepared in accordance with the method of A. Imam, et al. (Cancer Research 45, 263(1985)). First, lymphocytes are isolated from a cancer-associated lymph node excised from a cancer patient and then fused with mouse myeloma cells in the presence of polyethyleneglycol. From the supernatant of the hybridomas thus obtained, those producing an antibody positive to various cancer cell lines fixed with paraformaldehyde are selected by means of enzyme immunoassay, and cloned.

From the supernatant of the hybridomas, monoclonal antibodies are purified in the conventional manner [R.C. Duhamel, et al., J. Immunol. Methods, 31, 211(1979)] and labeled with a fluorescent substance. The reactivity with a living cancer cell line or with erythrocyte and leukocyte was detected by flow cytometry, whereby antibodies exhibiting reactivity with the living cancer cell line but not with erythrocyte and leukocyte are selected. In addition, the reactivity of antibodies with cancer cells isolated from the cancer tissue excised from a cancer patient is compared with the reactivity of the antibodies with normal cells isolated from non-cancer segment of the same tissue of the same patient, and the antibody which is bound to the cancer cells in a greater amount and does not react with normal cells or which shows reactivity as low as an antibody obtained from normal volunteer is selected.

A base sequence of a DNA encoding an antibody produced from the hybridoma selected as described above can be obtained, for example, in the following manner. In accordance with the guanidine thiocyanate-lithium chloride method [Casara, et al, DNA, 2, 329(1983)], mRNA is separated from the antibody-producing hybridoma and by using an oligo (dT) primer, its cDNA library is prepared. The cDNA thus obtained is then subjected to (dG) tailing. The antibody-encoding cDNA is amplified by the PCR method while using, as probes, poly C to be hybridized with the resulting dG tail and a consensus sequence of human antibody heavy-chain gene and light-chain gene. The terminal of the amplified DNA is made blunt. The DNA separated from an electrophoresis gel is inserted to a cloning vector such as pUC119, and the base sequence of the DNA is determined by the dideoxy method of Sanger et al [Proc. Natl. Acad. Sci. U.S.A. 74, 5463(1977)]. Based on this base sequence, the hybridoma having the above-described specific amino acid sequence can be selected.

The monoclonal antibody to be used in the present invention can also be prepared by genetic engineering technique.

The especially preferred monoclonal antibodies of the invention are those in which the heavy chain variable region and light chain variable region are represented by the amino acid sequences of SEQ. ID NOS. 7 and 8 of Sequence Listing, respectively. The base sequences encoding constant regions of the heavy and light chains are the same as those described in Nucleic Acids Research, 14, 1779(1986), The Journal of Biological Chemistry, 257, 1516(1982) and Cell, 22, 197(1980).

The antibody of the invention may be prepared by culturing the hybridoma producing the antibody of the invention in an eRDF, RPMI 1640 or the like medium containing fetal bovine serum. Alternatively, it may also be prepared by chemically synthesizing a gene in which DNAs encoding variable regions including the above-described specific hypervariable regions have been linked respectively with DNAs encoding the constant regions of heavy chains and light chains; inserting the gene into a known expression vector enabling the gene construction, for example, pKCR (ΔE)/H and pKCRD, which can be constructed from pKCRH2 [Mishina, et al., Nature, 307, 605(1984)] in the procedure shown in FIG. 1 or FIG. 2 of Japanese Patent Application Laid-Open No. Hei 5-304987; and causing them to express in a host such as CHO cells (Chinese Hamster ovary cells). For example, a heavy chain gene having each end added with a HindIII site is inserted into the HindIII site of pKCR (ΔE)/H, while a selective marker gene such as DHFR gene is inserted into the SalI site of this plasmid. On the other hand, a light chain gene having each end added with EcoRI site is inserted into the EcoRI site of pKCRD and then the DHFR gene is also inserted into the SalI site of this plasmid. Both plasmids are introduced into cells such as CHOdhfr-[Urlaub G. & Chasin L. A., Proc. Natl. Acad. Sci. U.S.A., 77, 4216(1980)] by the calcium phosphate method. The antibody can be obtained by selecting the antibody producing cells from the cells proliferated in an αMEM culture medium free of nucleotide. The antibody is purified by causing protein A, in the medium used for culturing of these cells, to adsorb to a column bound to a support such as cellulofine or agarose and then eluting the antibody from the column.

As the antibody, whole length antibody (whole antibody) or antibody fragment, or antibody derivative can be used. The term "antibody" as used herein embraces, as well as the whole antibody and antibody fragment (such as F(ab'), F(ab')2 and scFv (one-strand antibody)), antibody derivatives and modified antibodies. This term must be interpreted most broadly.

When the remedy for mammary cancer according to the present invention is administered to human beings, the human monoclonal antibody is advantageous because it is not a protein of a different animal.

No particular limitation is imposed on the kind of an antitumor substance to be used in the present invention. Examples include antitumor agents (anticancer agents) such as doxorubicin (adriamycin), daunomycin, vinblastine, cisplatin and 5-fluorouracil (5-FU), toxins such as lysine A and diphteria toxin, antisense RNA, and pharmacologically acceptable salts or derivatives thereof. These substances are available by purchasing a commercially available product or preparing in a known manner as needed.

As the above-described pharmacologically acceptable salts, salts with a pharmacologically acceptable polyvalent anionic substance such as citrates, tartrates and glutaminates, and salts with their derivatives are preferred.

The antibody and antitumor substance can be associated by a method of chemically binding the antibody to the antitumor substance, a method of encapsulating an antitumor substance in a liposome and then binding the antibody to the surface of the liposome or a method usable for those skilled in the art.

In the remedy for mammary cancer according to the present invention, the antitumor substance is preferably associated with the antibody by-binding the antibody to the surface of a liposome having the antitumor substance encapsulated therein.

Examples of the lipid constituting the liposome include, but not limited to, natural lecithins (such as egg yolk lecithin and soybean lecithin), phospholipids such as dipalmitoylphosphatidylcholine (DPPC), dimyristoylphosphatidylcholine (DMPC), distearoylphosphatidylcholine (DSPC), dioleoylphosphatidylcholine (DOPC), dimyristyolphosphatidylethanolamine (DMPE), dipalmitoylphosphatidylethanolamine (DPPE), dioleoylphosphatidylethanolamine (DOPE), dipalmitoylphosphatidic acid (DPPA), dipalmitoylphosphatidylglycerol (DPPG), and dimyristoylphosphatidic acid (DMPA), glycolipids such as glycosphingolipids and glyceroglycolipids, fatty acids, dialkyl dimethylammonium amphiphiles, polyglycerol alkyl ethers, polyoxyethylene alkyl ethers (Liposome Technology, 2nd edition, vol. 1, 141(1993)), alkyl glycosides, alkyl methyl glucamides, alkyl sucrose esters, dialkyl polyoxyethylene ethers and dialkyl polyglycerol ethers (Liposome Technology, 2nd edition, vol. 1, 141(1993)), and amphipathic block copolymers such as polyoxyethylene-polylactic acid (International Patent Publication No. 508831/1994). These lipids may be used either singly or in combination. They may be used in combination with a nonpolar substance such as cholesterol or a cholesterol derivative such as DC-cho1 (3β-[N-(N',N'-dimethylaminoethyl) carbamoyl] cholesterol) .

In the liposome, it is preferred to use, as a part of the lipid component, a lipid which has been maleimidated (which will hereinafter be called "maleimidated lipid") such as maleimidated phosphatidylethanolamine in order to bind a polyalkyleneglycol-containing compound and, if necessary, a protein such as antibody to the liposome. A ratio of the maleimidated lipid in the whole lipid is usually about 0.5 to 10 mole%.

Maleimidated phosphatidylethanolamine, for example, is available by the reaction between a maleimide-containing compound having reactivity with an amino group and the amino group of phosphatidylethanolamine (PE). This maleimide-containing compound may contain a residue such as caproyl, benzoyl, phenylbutyryl or the like group. Examples include N-(ε-maleimidocaproyloxy)succinimide, N-succinimidyl 4-(p-maleimidophenyl)butyrate, N-succinimidyl 4-(p-maleimidophenyl)propionate and N-(γ-maleimidobutyryloxy)succinimide. Examples of the PE usable in this reaction include dipalmitoylphosphatidylethanolamine (DPPE), dimyristoylphosphatidylethanolamine (DMPE), and dioleoylphosphatidylethanolamine (DOPE). Of these, DPPE is preferred. The liposome may contain as another lipid component a charge imparting substance such as stearylamine and dicetylphosphate. The liposome may exist as a fusion liposome having a portion or whole portion of virus incorporated therein, for example, a fusion liposome of Sendai virus and liposome.

As a typical liposome, a lipid composition containing, per mole of phosphatidylcholine, 0.3 to 1 mole, preferably 0.4 to 0.6 mole of cholesterol and 0.01 to 0.2 mole, preferably 0.02 to 0.1 mole, more preferably 0.02 to 0.05 mole of maleimidated phosphatidylethanolamine can be used. When phosphatidic acid is added, the lipid composition contains it in an amount of 0.4 mole or less, preferably 0.15 mole or less.

No particular limitation is imposed on the preparation process of the liposome. Any process usable by those skilled in the art can be employed. The liposome is able to take any form without particular limitation. For example, any one of multilamella liposome (MLV) formed by adding an aqueous solution to a thin lipid film attached to a glass wall and mechanically shaking the mixture; small unilamella liposome (SUV) available by ultrasonication, ethanol injection or French press; and large unilamella liposome (LUV) available by surfactant removal, reverse phase evaporation ("Liposome", by Junzo Sunamoto, et al., Nankodo, 1998), or extrusion of MLV from a membrane having a uniform pore size while applying pressure (Liposome Technology, 2nd edition, vol. 1, 141, 1993). The particle size of the liposome is, for example, about 300 nm or less, preferably 30 to 200 nm.

In the liposome, an antitumor substance is encapsulated. No particular limitation is imposed on the introducing method of the antitumor substance into the liposome and any method usable by those skilled in the art can be employed. For example, the substance may be encapsulated in the liposome by adding it as an aqueous solution upon formation of the liposome. Alternatively, it is possible to form a density gradient such as pH gradient inside and outside of a vesicle after formation of the liposome, and encapsulate an ionizable antitumor substance to the inside of the liposome with this potential as a driving force (Cancer Res., 49, 5922, 1989; BBA, 455, 269, 1976).

The antibody may be attached to the surface of the liposome by a method of binding a hydrophobic substance to a purified antibody and then inserting it into the liposome, a method of crosslinking phosphatidylethanolamine and the antibody via glutar, or the like. Preferred is a method of adding a thiol group to the antibody and then causing the maleimide group of the liposome to react with the thiolated antibody, thereby modifying the liposome with the antibody. The addition of a thiol group to the antibody can be carried out by causing a compound such as N-succinimidyl-3-(2-pyridyldithio)propionate (SPDP) (Carlsson, J., et al., Biochem. J., 173, 723, 1978), or iminothiolane, or mercaptoalkyl imidate (Traut, R.R., et al., Biochemistry, 12, 3266, 1973) ordinarily employed for the thiolation of a protein to react with the amino group of the antibody.

It is also possible to cause a sulfur-containing group derived from the antibody, that is, an endogenous dithiol group of the antibody to react with the maleimide group. Use of this endogenous dithiol group is preferred in view of maintaining the activity of the antibody. The endogenous dithiol group of the antibody, after reduced into a thiol group, can be caused to react with the maleimide group. For example, when IgG is used, a thiol group of Fab' which is available by conversion of the IgG into an F(ab')₂ fragment by using an enzyme such as pepsin, followed by reduction by using dithiothreitol, can be utilized in the antibody-liposome reaction (Martin, F.J. et al., Biochemistry, 20, 4229, 1981). When IgM is used, a thiol group of an Fc region of IgMs obtained by reducing the J chain under mild conditions in accordance with the method of Mirror et al. (J. Biol. Chem., 257, 286, 1965) can be utilized for the antibody-liposome reaction. When the GAH antibody as described in Japanese Patent Application Laid-Open No. Hei 5-304987 is used, an F(ab')₂ fragment is preferably used. Binding of a protein such as a thiol-added antibody to a maleimide-containing liposome can be accomplished by reacting them in a neutral buffer (pH 6.5 to 7.5) for 2 to 16 hours.

A liposome having a surface bound to a polyalkyleneglycol-portion-containing compound is preferred. As the polyalkyleneglycol, polyethyleneglycol (PEG) and polypropyleneglycol can be used, with polyethyleneglycol being preferred. Polyethyleneglycol, if it is employed, having a molecular weight of from about 2,000 to 7,000 daltons, preferably about 5,000 daltons can be used.

A liposome preferably has a polyalkyleneglycol-portion-containing compound bound, via a thioether bond, to the maleimidated lipid on the surface of the liposome. In this case, the liposome to which polyalkyleneglycol has been bound can usually be prepared by introducing a thiol group into a polyalkyleneglycol-portion-containing compound and then causing the resulting compound to react with the maleimide group of the liposome. Examples of the polyalkyleneglycol-portion-containing compound include compounds having a polyethyleneglycol group and at the same time, having, at the terminal of the compound, a compound which can be thiolated or a compound having a mercapto group, more specifically, compounds having a polyalkyleneglycol group bound to triazine and these compounds whose triazine has been substituted with an amino acid or the like. In this case, compounds may have two polyalkyleneglycol groups (double strand).

For the preparation of a polyalkyleneglycol-portion-containing compound having a thiol group introduced therein, when polyethyleneglycol is used as the polyalkyleneglycol, a method of subjecting monomethoxypolyoxyethyleneamine and a thiolcarboxylic acid to dehydration/condensation; a method of introducing a pyridyldithiopropionyl group into monomethoxypolyoxyethyleneamine by using SPDP, followed by reduction; a method of introducing a thiol group into monomethoxypolyoxyethyleneamine by using iminothiolane; a method of coupling an active ester of monomethoxypolyoxyethylenecarboxylic acid with a thiol-amine; and a method of condensing a polyethyleneglycol triazine derivative with a thiol-amine. More specifically, 2,4-bis(polyethyleneglycol)-6-chloro-2-triazine (activated PEG2 (product of Seikagaku Corporation)) can be caused to react with cystine, followed by reduction into cysteine-bound activated PEG2.

No particular limitation is imposed on the amount of the polyalkyleneglycol-portion-containing compound to be bound to the maleimidated lipid in the liposome. Although it may be caused to react with an excess amount of the remaining maleimidated lipid, the amount of the polyalkyleneglycol is preferably from about 0.28 to 0.90 mole%, more preferably from about 0.28 to 0.56 mole% based on the whole lipid, and from about 15 to 50 mole%, more preferably from about 15 to 30 mole% based on the maleimidated lipid, and from about 0.44 to 1.45 mole%, more preferably from about 0.44 to 0.89 mole% based on DPPC.

In the preferred embodiment of the present invention, a liposome having the antibody and the polyalkyleneglycol-portion-containing compound bound each other is used. This liposome may be prepared in the following manner. First, a thiolated antibody is caused to react with a maleimide-containing liposome in a neutral buffer. For example, this reaction may be effected so that an amount of the antibody bound to the liposome would be 0.5 to 5.3 mg, preferably 0.5 to 4.5 mg, more preferably 1.2 to 2 mg per-100 mg of a whole lipid constituting the liposome. Described specifically, about 0.1 mole% to 2 mole%, preferably 0.1 to 1.6 mole%, more preferably 0.4 to 0.7 mole% of the thiolated antibody may be caused to react with 1 mole of the maleimide group (maleimidated lipid). Then, a thiolated polyalkyleneglycol-portion-containing compound is caused to react with the remaining maleimide group, whereby a liposome having the antibody and polyalkyleneglycol-portion-containing compound bound each other can be prepared. More specifically, the liposome having the antibody and polyalkyleneglycol-portion-containing compound bound each other can be prepared by adding 15 mole% to 50 mole%, preferably 15 to 30 mole% (0.28 to 0.90 mole%, preferably 0.28 to 0.56 mole% based on the whole lipid, and when DPPC is used, 0.44 to 1.45 mole%, preferably 0.44 to 0.89 mole% based on DPPC) of the thiolated polyalkyleneglycol-portion-containing compound to 1 mole of the maleimidated lipid group.

An antitumor-substance-containing liposome having an antibody bound thereto can be formulated as a medicament by a known manner, for example, a dehydration method (International Patent Publication No. 502348/1990), a method of adding a stabilizer and using the mixture as a liquid preparation (Japanese Patent Application Laid-Open No. Sho 64-9331), a method of freeze drying (Japanese Patent Application Laid-Open No. Sho 64-9931), or the like method. For the treatment of mammary cancer, the preparation can be administered to patients through intravascular administration, topical administration or the like. A dose can be appropriately selected depending on a type of an antitumor substance serving as an effective ingredient. For example, when a liposome having doxorubicin encapsulated therein is administered, 50 mg/kg or less, preferably 10 mg/kg or less, more preferably 5 mg/kg or less can be administered as an amount of an effective ingredient.

### EXAMPLES

The present invention will hereinafter be described in detail by Examples. It should however be borne in mind that the present invention is not limited to or by them provided that its essence is not exceeded.

### Example 1: Reactivity of GAH antibody with mammary cancer tissue

A GAH antibody as described in Japanese Patent Application Laid-Open No. Hei 5-304987 (Examples 1, 2 and 3) was labeled with a biotinylating reagent (product of Amersham Bioscience). After a paraffin section of human mammary cancer tissue was de-paraffinized and then blocked by dipping it in a 5%-BSA/PBS solution at room temperature for 1 hour, the resulting section was caused to react with 100 µg/ml of a biotinylated GAH antibody solution at 37°C for 2 hours. The section was washed with PBS and caused to react with 4 µg/ml of a PerCP (peridinin chlorophyll protein) labeled streptavidin solution (product of Becton/Dickinson) for 30 minutes under ice cooling while blocking light. The reactivity of the GAH antibody with the mammary cancer tissue section was detected as red fluorescence of PerCP having an emission wavelength of 680 nm at an excitation wavelength of 490 nm by using a fluorescence microscope. As a result of judging the reactivity of the GAH antibody from its intensity of specific red fluorescence and distribution, it has been confirmed that Of 11 cases of mammary cancer tissue, positive reaction occurred in 5 cases.

The mammary cancer cell lines MDA-MB231, MDA-435 and MDA-MB468 (each, product of Dainippon Pharmaceutical) were cultured and each cell was implanted subcutaneously to a nude mouse (5 week old, male, purchased from Nippon CLEA). When the tumor became about 1 cm³ as a result of proliferation, it was excised and by paraffin embedding, its tissue section was prepared. The reactivity of the GAH antibody with each of these mammary cancer tissue sections was analyzed in a similar manner to that employed for the analysis of the reactivity with the human mammary cancer tissue section. As a result, of the three mammary cancer lines, the positive reaction was recognized from MDA-MB231.

### Example 2: Proliferation inhibitive effect of GAH-antibody-bound liposome for mammary cancer cell line

In accordance with the method as described in WO00/64413 (Example 1), a liposome having doxorubicin (DXR) (product of Kyowa Hakko Kogyo) encapsulated therein was prepared. To the resulting liposome, a thiolated GAH antibody and thiolated polyethyleneglycol (PEG) were attached successively, whereby an antibody-bound liposome was prepared. In a similar manner except that the antibody was not attached, an antibody-unbound liposome was prepared.

The mammary cancer cell line MDA-MB231 whose reactivity with the GAH antibody had been confirmed was inoculated to a 96-well plate at a density of 5 × 10³/well and cultured for 2 days on an e-RDF medium (product of GIBCO BRL) added with 10% FBS. Then, the culture supernatant was removed and 100 µl/well of the GAH antibody-bound liposome or antibody-unbound liposome having a concentration of 5 µg/ml in terms of the amount of DXR was added to each of 9 wells. After reaction at 37°C for 1 hour, each liposome solution was removed and culturing was continued by adding an e-RDF medium added with 10% FBS. On Day 5, MTT (tetrazolium salt, 3-(4,5-dimethyl-thiazolyl-2-yl)-2,5-diphenyltetrazolium bromide) assay (Green, L.M., et al. J. Immunol. Methods 70: 257-268, 1984) was performed in order to compare a survival rate of cells. The formazan formed by a mitochondrial dehydrogenase enzyme in living cells was dissolved in 0.04N-HCl-added isopropyl alcohol and the absorbance at 550 nm was measured. With a survival rate in the well having a liposome addition concentration of 0 taken as 100%, a survival rate of cells in the well added with each of the antibody-bound liposome and antibody-unbound liposome was calculated. The results are illustrated in FIG. 1. It has been recognized that compared with the antibody-unbound liposome, the GAH-antibody-bound liposome has a more potent effect for preventing proliferation of mammary cancer cells.

### INDUSTRIAL APPLICABILITY

The present invention can provide a remedy for mammary cancer having high therapeutic effects on mammary cancer, as well as gastric cancer and colorectal cancer, by making use of the specific reactivity of an antibody.

The present application was filed, claiming priority from Japanese Patent Application 2001-224596.

## Claims

1. A remedy for mammary cancer comprising a human monoclonal antibody having amino acid sequences of SEQ. ID. NOS. 1, 2 and 3 of Sequence Listing in hypervariable regions of a heavy chain and amino acid sequences of SEQ. ID. NOS. 4, 5 and 6 of Sequence Listing in hypervariable regions of a light chain; and an antitumor substance associated with the antibody.

2. A remedy for mammary cancer of Claim 1, wherein the monoclonal antibody has a heavy chain variable region containing an amino acid sequence of SEQ. ID No. 7 of Sequence Listing and a light chain variable region containing an amino acid sequence of SEQ. ID No. 8 of Sequence Listing.

3. A remedy for mammary cancer of Claim 1 or 2, wherein the antitumor substance has been associated with the antibody by binding the antibody to the surface of a liposome having the antitumor substance encapsulated therein.

4. A mammary cancer of Claim 3, wherein the antibody has been bound to the surface of the liposome by attaching via a thioether group the antibody to the liposome having a lipid end partially maleimidated.

5. A remedy for mammary cancer of Claim 4, wherein 0.1 to 2 mole% of the antibody has been bound to 1 mole of the maleimidated lipid.

6. A remedy for mammary cancer of Claim 4 or 5, wherein the antibody has been bound to the surface of the liposome by causing the maleimide-containing liposome to react with a sulfur-containing group derived from the antibody to form a thioether bond.

7. A remedy for mammary cancer of any one of Claims 3 to 6, wherein to the surface of the liposome, a compound containing a polyalkyleneglycol portion has been bound further.

8. A remedy for mammary cancer of Claim 7, wherein 15 to 50 mole% of the compound containing a polyalkyleneglycol portion has been bound to 1 mole of the maleimidated lipid contained in the liposome.

9. A remedy for mammary cancer of Claim 7 or 8, wherein the compound containing a polyalkyleneglycol portion has been bound to the surface of the liposome by causing the maleimide group of the maleimidated lipid to react with the compound containing a polyalkyleneglycol portion added with a thiol group.

10. A remedy for mammary cancer of any one of Claims 7 to 9, wherein the polyalkyleneglycol portion is a polyethyleneglycol portion.

11. A remedy for mammary cancer of Claim 10, wherein the compound containing a polyalkyleneglycol portion has two polyethyleneglycol portions.

12. A remedy for mammary cancer of Claim 10 or 11, wherein the polyethyleneglycol portion has a molecular weight of from 2,000 to 7,000 daltons.

13. A remedy for mammary cancer of any one of Claims 1 to 12, wherein the antibody is an F(ab')₂ fragment.


SEQUENCE LISTING

<110> 三菱ウェルファーマ株式会社(MITSUBISHI PHARMA CORPORATION)

<120> 乳癌治療薬

<130> 02031W00

<140>
<141>

<150> JP P2001-224596
<151> 2001-07-25

<160> 8

<210> 1
<211> 9
<212> PRT
<213> Homo sapiens

<400> 1
Ile Ser Ser Cys Gly Phe Tyr Trp Asn
 1               5

<210> 2
<211> 12
<212> PRT
<213> Homo sapiens

<400> 2
Ile Gly Tyr Ile Tyr Tyr Ser Gly Ser Thr Tyr Tyr

1               5                   10

<210> 3
<211> 9
<212> PRT
<213> Homo sapiens

<400> 3
Ser Thr Arg Leu Arg Gly Ala Asp Tyr
1               5

<210> 4
<211> 17
<212> PRT
<213> Homo sapiens

<400> 4
Lys Ser Ser Gln Ser Val Leu Tyr Asn Ser Asn Asn Lys Lys Tyr Leu
1               5                   10                  15
Ala

<210> 5
<211> 7
<212> PRT
<213> Homo sapiens

<400> 5
Trp Ala Ser Thr Arg Glu Ser
1               5

<210> 6

<211> 9
<212> PRT
<213> Homo sapiens

<400> 6
Gln Gln Tyr Tyr Ser Thr Pro Trp Thr
1               5

<210> 7
<211> 119
<212> PRT
<213> Homo sapiens

<400> 7
Gln Val Gln Leu Gln Glu Ser Gly Pro Gly Leu Val Lys Pro Ser Gln
1               5                   10                  15
Thr Leu Ser Leu Thr Cys Thr Val Ser Gly Gly Ser Ile Ser Ser Cys
            20                  25                  30
Gly Phe Tyr Trp Asn Trp Ile Arg Gln His Pro Gly Lys Gly Leu Glu
        35                  40                  45
Trp Ile Gly Tyr Ile Tyr Tyr Ser Gly Ser Thr Tyr Tyr Asn Pro Ser
    50                  55                  60
Leu Lys Ser Arg Val Thr Ile Ser Leu Asp Thr Ser Lys Ser Gln Phe
65                  70                  75                  80
Ser Leu Lys Leu Ser Ser Leu Thr Ala Ala Asp Thr Ala Val Tyr Tyr
                85                  90                  95
Cys Ala Arg Ser Thr Arg Leu Arg Gly Ala Asp Tyr Trp Gly Gln Gly
            100                 105                 110
Thr Met Val Thr Val Ser Ser
        115

<210> 8

<211> 114

<212> PRT

<213> Homo sapiens

<400> 8

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Val Leu Tyr Asn
            20                  25                  30

Ser Asn Asn Lys Lys Tyr Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Tyr Ser Thr Pro Trp Thr Phe Gly Gln Gly Thr Lys Val Glu Ile
            100                 105                 110

Lys Arg